# EUROPEAN PATENT SPECIFICATION

(11) **EP 0 826 672 B1**
(45) Date of publication and mention of the grant of the patent: **15.06.2005**
(21) Application number: 97306380.3
(22) Date of filing: 21.08.1997
(51) Int. Cl.: C07D 213/78

(54) **Process for the preparation of azine-carboxylic acid chlorides**
Verfahren zur Herstellung von Azincarbonsäurechloriden
Procédé pour la préparation de chlorides d azine acides carboxyliques à partir de trichloromethylazines

(30) Priority: 23.08.1996 EP 96113531; 07.11.1996 EP 96117863
(43) Date of publication of application: 04.03.1998
(73) Proprietor: BASF AKTIENGESELLSCHAFT, 67056 Ludwigshafen (DE)
(72) Inventor: Knell, Marcus, 55270 Schwabenheim (DE); Brink, Monika, 55218 Ingelheim (DE)
(74) Representative: Langfinger, Klaus Dieter, Dr.

(56) References cited:
- EP-A- 0 091 022
- EP-A- 0 646 566
- US-A- 1 921 767
- US-A- 1 965 556
- US-A- 2 856 425
- US-A- 5 166 352
- CHEMICAL ABSTRACTS, vol. 124, no. 9, 26 February 1996 Columbus, Ohio, US; abstract no. 116872u, TEJIMA T. & KAWAHARA S.: "Preparation of carboxylic acid chlorides" page 1219; column 1; XP002047950 & JP 07 291 891 A (TOKUYAMA CORP.) 7 November 1995

## Description

### BACKGROUND OF THE INVENTION

The present invention relates to a process for the preparation of azinyl acid chloride compounds from a trichloromethylazine in which the trichloromethylazine is treated with an acid, which is capable of forming an acid chloride that can be distilled off under reduced pressure in the course of the reaction, in the presence of an acidic catalyst.

Azinyl acid chloride compounds are suitable intermediates for the preparation of a broad variety of compounds which are useful as agrochemicals, pharmaceuticals or liquid crystals. In particular, they are key intermediates in the preparation of herbicidal pyridinecarboxamides which are described for example in EP 0 447 004 A.

US patent no. 3,875,226 discloses a process wherein trichloromethyl compounds are treated with sulfur dioxide in the presence of a Lewis acid to form acid chloride compounds and thionyl chloride.

However, this process is hardly applicable in technical scale since sulfur dioxide is gaseous under normal conditions and has to be handled under cooling and/or under pressure, these conditions are not applicable in the large scale production.

The European patent application EP 0 646 566 A suggests to hydrolyze trichloromethyl azines with water in the presence of chlorinated hydrocarbons and a Lewis acid.

However, this process causes problems with respect to the dosing rate and the exact equimolar dosing of water. Any excess of water will cause hydrolysis of the desired acid chloride compound and therefore reduce the yields.

Moreover, in these days using chlorinated hydrocarbons is not desired because of environmental problems, and the amount of solvent used in the prior art procedure is high. Furthermore, the reaction time needed using water/1,2-dichloroethane is very long (24 h).

EP 0 091 022 discloses the preparation of isoxazole-5-carboxylic acid from 5-trichloromethylisoxazole using trichloroacetic acid and antimony pentachloride or ferric chloride as Lewis acids.

However, there is no hint that this process could be applied to other trichloromethylazines and other carboxylic acid. Whereas the reaction using antimony pentachloride was completed within 2 hours, ferric chloride took 8 hours. One of the disadvantages of this process is that the expensive antimony pentachloride is poisonous and therefore this process cannot be used in technical scale. Moreover, only low yields are achieved if antimony pentachloride is used for the preparation of azinoyl chlorides.

The German patent application DE 30 04 693 discloses a process for simultaneous preparation of aromatic sulfonyl halides and benzoyl halides by the reaction of aromatic sulfonic acids with trichloromethyl arenes.

However, the separation of these products requires high sophisticated distillation techniques.

JP 7291891 provides a method for preparing benzoylchloride by reacting an aliphatic acid with benzotrichloride in the presence of an inorganic iron, aluminium or zinc salt.

More precisely US 1,921,767 discloses the reaction of benzotrichloride with acetic acid at atmospheric pressure in the presence of catalytic amounts of cobalt chloride or zinc chloride.

Furthermore US 1,965,556 discloses the preparation of benzoylchloride by reacting benzotrichloride with acetic acid in the presence of a strong mineral acid like sulphuric acid.

Moreover US 2,856,425 teaches the preparation of benzoylchlorides or related compounds by reacting the respective trichloromethyl derivatives with titanium oxide or vanadium pentoxide. The resulting volatile metal chlorides and/or metal oxyhalides are distilled off.

According to US 5,166,352 optionally substituted pyridine carboxylic acid chlorides are prepared by reacting the respective trichloromethyl pyridine in the vapour phase at a temperature of about 250 to about 450 °C with aluminium oxide.

The problem to be solved was to provide a process for the preparation of azinyl acid chlorides in high yields, which avoids solvents causing environmental problems and long reaction times.

### SUMMARY OF THE INVENTION

Surprisingly, it was found that azinyl acid chloride compounds of formula I, 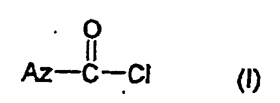 wherein
Az represents a 6-membered heterocyclic group with at least one nitrogen atom, being optionally substituted by one or more halogen atoms, nitro, cyano, C₁-C₆-alkyl, C₁-C₆-alkoxy, 4-C₁-C₆-alkyl-cyclohexyl or C₁-C₆-haloalkyl,
can be easily prepared in high yields with the aid of a process which comprises heating a trichloromethylazine of formula II

Az-CCl₃ (II)

wherein Az has the meaning given
with an acid of formula III

R¹-X-OH (III)

wherein
R¹ represents a C₁₋₆alkyl or C₁₋₆haloalkyl group, and
X represents CO or SO₂,
that forms the acid chloride of the acid of formula III, which can be distilled off during the reaction at reduced pressure;
in the presence of a catalyst.

. It is, therefore, an object of the present invention to provide an efficient new process for the preparation of azinoyl chloride compounds.

Another aspect of the invention is the use of the azinoyl chlorides obtained according to the process of the present invention for the preparation of aryloxy azinyl carboxamides.

Other objects and advantages of the present invention will be apparent to those skilled in the art from the following description and the appended claims.

### DETAILED DESCRIPTION OF THE INVENTION

In general terms, unless otherwise stated herein, the term optionally substituted azinyl group, as used herein with respect to Az refers to a 6-membered heterocyclic group with at least one nitrogen atom, in particular to a pyridine or pyrimidine group being optionally substituted by one or more halogen atoms, nitro, cyano, C₁₋₆ alkyl, C₁₋₆ alkoxy, 4-C₁₋₆ alkyl-cyclohexyl, C₁₋₆ haloalkyl groups.

As a rule heteroaromatic groups are preferred, which are substituted by at least one electron-withdrawing group, in particular by one or more halogen atoms, nitro, cyano or haloalkyl groups.

.In a particularly preferred embodiment Az represents an optionally substituted pyridyl group of formula V, 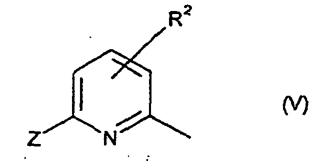 in which
R² represents a hydrogen or halogen atom or an C₁₋₆ alkyl or C₁₋₆ haloalkyl group, and
Z represents a halogen atom.

In general terms, unless otherwise stated herein, the term alkyl or haloalkyl as used herein with respect to a radical or moiety refers to a straight or branched chain radical or moiety. Preferably an alkyl or haloalkyl moiety has from 1 to 3 carbon atoms.

A preferred alkyl moiety is an ethyl or especially a methyl group

Preferred haloalkyl groups are poly- or perhaloginated alkyl groups of formula -(CX₂)ₙ-Y, in which n is an integer of 1 to 6, in particular 1 to 3, X represents fluorine or chlorine and Y is hydrogen or X. A preferred polyhaloginated alkyl moiety is a pentafluoroethyl, pentachloroethyl or especially a difluoro- or a trifluoromethyl group or a dichloro- or a trichloromethyl group.

Optionally substituted moieties may be unsubstituted or have from one up to the maximal possible number of substituents. Typically, 0 to 2 substituents are present.

Further preferred embodiments of the process according to the present invention is a process wherein:
(a) A process, wherein Az represents an azinyl group which is substituted by one halogen atom and optionally substituted by one C₁₋₆ alkyl or C₁₋₆ haloalkyl group, preferably a substituted pyridyl group of formula V, 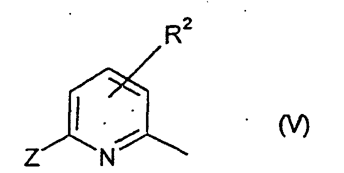 in which
   R² represents a hydrogen or halogen atom or an C₁₋₆ alkyl or C₁₋₆ haloalkyl group, and
   Z represents a halogen atom, in particular a 6-halopyrid-2-yl group.
(b) A process, wherein R' represents a methyl group being optionally substituted by one or more chlorine atoms.
(c) A process, wherein the acidic catalyst is selected from sulfuric acid, FeCl₃ and ZnCl₂.
(d) A process, wherein a reaction mixture essentially consisting of the trichloromethylazine of formula II, the acid of formula III and the acidic catalyst is heated and the acid chloride of formula IV,

   R¹-X-Cl (IV)

   wherein R¹ and X are as defined above,
   formed in course of the reaction is distilled off under reduced pressure.
(e) A process, wherein 1 mole of trichloromethylazine of formula II is treated with 0.4 to 1.2 moles of an acid of formula III.
(f) A process, wherein X represents SO₂ and the acidic catalyst is sulfuric acid with a content of less than 5 % by weight of water.
(g) A process, wherein 1 mole of trichloromethylarene of formula II is treated with the acid of formula III in the presence of 0.01 to 0.10 moles of the acidic catalyst.

The reaction is carried out at a temperature between ambient temperature and the reflux temperature of the reaction mixture, preferably at elevated temperature, especially at reflux temperature, preferably between 75 and 160 °C, in particular between 85 and 130 °C.

Another aspect of the invention is:
The use of a compound of formula I for the preparation of aryloxy-heteroarylcarboxamides of formula VI 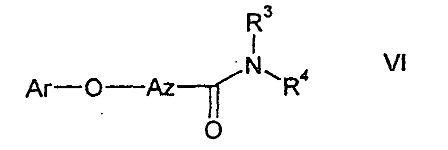 in which
   Az represents a 6-membered heterocyclic group with at least one nitrogen atom, being optionally substituted by one C₁-C₆ alkyl or C₁-C₆ haloalkyl group, preferably an optionally substituted pyridyl group,
   Ar represents a phenyl group being substituted by at least one halogen atom or a C₁-C₆ haloalkyl or C₁-C₆ haloalkoxy group, in particular a 3-trifluoromethyl-phenyl group,
   R³ represents a hydrogen atom or a C₁-C₆ alkyl group, preferably a hydrogen atom, and
   R⁴ represents a phenyl group being substituted by at least one halogen atom or a C₁-C₆ haloalkyl or C₁-C₆ haloalkoxy group, in particular a 4-fluorphenyl group,
wherein
(a) the mono-halogenated azinoyl chloride of formula I being prepared from a mono-halogenated azinyltrichloromethane of formula II according to any of the claims 1 to 7,
(b) is reacted with an with an amine of formula VII

   HNR³R⁴ VII

   in which R³ and R⁴ are as defined above, optionally in the presence of an inert solvent and/or a base, and
(c) the resulting mono-halogenated azinylcarboxamide is reacted with an aromatic or heteroaromatic hydroxyl compound of formula VIII,

   Ar-OH VIII

   in which Ar has the meaning given, in the presence of a base, in particular wherein the halogenated azinyl acid chloride of formula I obtained according to any of the claims 1 to 7 is reacted with an amine of formula VII without further purification.

As a rule the reaction is carried out under reduced pressure in order to facilitate the distillation of the compound of formula IV formed during the reaction. Most preferred the reaction is carried out at pressures between 20 and 400 mbar, in particular between 25 and 250 mbar.

In a particularly preferred embodiment of the process according to the invention, 1 equivalent of the trichloromethylazine of formula II, preferably in which Az is a substituted pyridine group, in particular Nitrapyrin, is mixed with 0.05 to 0.15 equivalents of the catalyst, in particular FeCl₃ and heated to 80 to 150°C, in particular 110 to 130 °C. Then 0.8 to 1.2 equivalents of the acid of formula III, wherein X represents CO, in particular chloroacetic acid, dichloroacetic acid or trichloroacetic acid are dosed to this reaction mixture, which is kept at reduced pressure. The corresponding acyl chloride of formula IV is distilled off until the reaction is completed.

Under this preferred reaction condition the reaction is as a rule completed within 1 to 5, in particular 1.5 to 4 hours.

In another particularly preferred embodiment of the process according to the invention 1 equivalent of the trichloromethylazine of formula II, preferably in which Az is a substituted pyridine group, in particular Nitrapyrin, is mixed with 0.01 to 0.05 equivalents of the catalyst, in particular H₂SO₄ and heated to 80 to 150°C, in particular 110 to 130 °C. Then 0.2 to 0.8 equivalents, the acid of formula III, wherein X is SO₂, in particular of methanesulfonic acid, are dosed to this reaction mixture, which is kept at reduced pressure. The corresponding alkanesulfonylchloride is distilled off until the reaction is completed.

If the reaction is carried out with equimolar amounts of heterocyclic trichloromethylazine of formula II and alkanesulfonic acid of formula III, tar-like by-products may be obtained which lower the achievable yields and cause troubles during the purification procedure. Therefore, using an excess of the trichloromethylazine can be advantageous, since this avoids the formation of these by-products.

In a particularly preferred embodiment of this invention an excess of 1 to 4 equivalents of Nitrapyrin is reacted with 1 mole of methanesulfonic acid in the presence of 0.01 to 0.05 moles of H₂SO₄ (96 to 99 % by weight).

Under this preferred reaction conditions the reaction is as a rule completed within 0.25 to 5, in particular 0.3 to 3 hours.

The remaining azinoyl chloride can be used as intermediate for the preparation of the desired end-products without further purification. It is also possible to purify them using standard procedures, as for example crystallization or distillation, in particular by distillation under reduced pressure, in particular at pressures between 1 and 100 mbar.

The novel process enables to carry out the production of azinyl acid chlorides in technical scale and high yields using cheap, ready-available educts. Moreover, the acids of formula III which are used as reagents in the new procedure can be recycled by addition of water to the corresponding acid chloride formed during the reaction. Thus, only a small amount of reagent is needed for the new procedure.

In order to facilitate a further understanding of the invention, the following illustrative examples are presented.

### Example 1

### Preparation of 2-chloro-6-pyridinecarbonylchloride

### [Az = 2-chloro-6-pyridyl in the compound of formula I]

A mixture of 200 mmol Nitrapyrin (2-chloro-6-trichloromethylpyridine) with the given amount of the catalyst is heated to temperatures between 90 and 130 °C. 200 mmol of the organic acid was dosed into the mixture under reduced pressure. The organic acid chloride formed by the reaction was distilled off during the reaction time. The reaction was monitored by GC analysis. The product is obtained by conventional work-up and distillation under reduced pressure as colorless crystals and shows the following physical properties:
mp 74-75°C, bp 80°C/2.6 Pa
¹H-NMR (DMSO, 300 MHz): δ (ppm) = 8.11 (m, 2 H, 3-, 5-CH), 7.80 (m, 1H, 4-CH)
¹³C-NMR (DMSO): δ (ppm) = 164.7 (q, COCI), 150.2 (q, 6-C), 148.8 (q, 2-C), 141.0 (t, 4-C), 127.9 (t, 3-C), 124.0 (t, 5-C).

The results of the experiments are given in the following table, in which the following abbreviations have been used:

| | | | |
|---|---|---|---|
| CPA | 6-chloropyridyl-2-carboxylic acid | AA | acetic acid |
| DCAA | dichloroacetic acid | CAA | chloroacetic acid |
| TCAA | trichloroacetic acid | PA | propionic acid |

| **Organic acid** | **Catalyst** | **mol%** **Catalyst** | **T (°C)** | **p (mbar)** | **t (h)** | **Yield** **(area%)** | **Yield CPA** **(area%)** |
|---|---|---|---|---|---|---|---|
| AA | FeCl₃ | 20 | 110 | 700 | 8 | 52 | 3 |
| CAA | FeCl₃ | 20 | 120 | 180 | 1,5 | 87 | - |
| DCAA | FeCl₃ | 10 | 115 | 170 | 4 | 85 | - |
| TCAA | FeCl₃ | 10 | 120 | 180 | 2 | 95 | - |
| TCAA | ZNCl₂ | 10 | 120 | 150 | 3 | 41 | - |
| PA | FeCl₃ | 10 | 120 | ambient | 10 | 46 | 25 |
| PA | ZNCl₂ | 10 | 120 | ambient | 10 | 30 | 15 |

### Example 2

### Preparation of 2-chloro-6-pyridinecarbonylchloride

### [Az = 2-chloro-6-pyridyl in the compound of formula I]

A mixture of the given amount of Nitrapyrin (NP, 2-chloro-6-trichloromethylpyridine) with 0.005 moles of the catalyst is heated to temperatures between 125 and 140 °C. 0.1 mol of methanesulfonic acid was dosed into the mixture under reduced pressure. The methanesulfonylchloride formed by the reaction was distilled off during 0.5 to 2 hours. The reaction was monitored by GC analysis. The product is obtained as a mixture of unreacted NP, which has been used in excess, and the desired product. This mixture can be used without further purification for the preparation of herbicidal pyridinecarboxamides. The product is obtained by distillation as colorless crystals and shows the following physical properties
mp 74-75°C, bp 80°C/2.6 Pa
¹H-NMR (DMSO, 300 MHz): δ (ppm) = 8.11 (m, 2 H, 3-, 5-CH), 7.80 (m, H, 4-CH).
¹³C-NMR (DMSO): δ (ppm) = 164.7 (q, COCI), 150.2 (q, 6-C), 148.8 (q, 2-C), 141.0 (t, 4-C), 127.9 (t, 3-C), 124.0 (t, 5-C).

The results of the experiments are given in the following table, in which the yields have been determined from the mixture obtained consisting of NP and the product:

**Table 2**

| **Example** | **NP** **(mol)** | **Catalyst** | **T** **(°C)** | **p** **(mbar)** | **Yield (% of th.)** |
|---|---|---|---|---|---|
| **2a)** | 0.2 | FeCl₃ | 130 | 100-50 | 78 |
| **2b)** | 0.2 | FeCl₃ | 135 | 50-45 | 88 |
| **2c)** | 0.2 | H₂SO₄ | 135 | 50-45 | 92 |
| **2d)** | 0.2 | H₂SO₄ | 135 | 50-40 | 90 |
| **2e)** | 0.2 | H₂SO₄ | 135 | 40 | 92 |
| **2f)** | 0.4 | H₂SO₄ | 135 | 40 | 97 |

### Preparation of 2-chloro-6-pyridinecarbonylchloride

A mixture of 200 mmol Nitrapyrin with 10 mmol SbCl₅ is heated to 120 °C. 200 mmol of the trichloroacetic acid was dosed into the mixture under ambient pressure. The trichloroacetyl chloride formed by the reaction was distilled off during 15 hours. The reaction was monitored by GC analysis. GC showed 9% product and 4 % 6-chloropyrid-2-ylcarboxylic acid.

### Use Example

### Preparation of N-(4-fluorophenyl) 6-(3-trifluoromethylphenoxy)-pyrid-2-ylcarboxamide

The crude product obtained according to Example 2e) is diluted with toluene (200 ml) and added to 4-fluoroaniline (250 mmol) at 65 °C. Subsequently the mixture is heated for 1 hour to 100 °C. The mixture is then cooled to 20 °C, washed with dilute hydrochloric acid and stripped to give an oil consisting essentially of N-(4-fluorophenyl) 6-chloropyrid-2-ylcarboxamide and unreacted nitrapyrin. The unreacted nitrapyrin is distilled off under reduced pressure. The obtained crude product (49,5 g, 87%) is diluted with toluene (200ml) and added without further purification to a mixture of potassium carbonate (210 mmol), 3-trifluoromethylphenol (200 mmol) and dimethylacetamide (120 ml). The toluene is distilled off and the reaction mixture is heated to 160 °C for 4 hours. The solvent is distilled off and the residue is diluted with toluene. The mixture is washed with NaHCO₃, dried and concentrated. The residue is recrystallized from methanol to give the title compound (58,2g, 85%) mp 105-107 °C.

## Claims

1. A process for the preparation of azinyl acid chloride compounds of formula I, 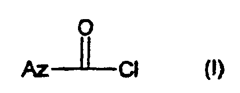 wherein
Az represents a 6-membered heterocyclic group with at least one nitrogen atom, being optionally substituted by one or more halogen atoms, nitro, cyano, C₁-C₆-alkyl, C₁-C₆-alkoxy, 4-C₁-C₆-alkyl-cyclohexyl or C₁-C₆-haloalkyl,
which comprises heating a trichloromethylazine of formula II
Az-CCl₃ (II)
wherein Az has the meaning given,
with an acid of formula III
R¹-X-OH (III)
wherein
R¹ represents a C₁-C₆-alkyl or C₁-C₆-haloalkyl, and
X represents CO or SO₂,
that forms the acid chloride of the acid of formula III, which can be distilled off during the reaction at reduced pressure;
in the presence of an acidic catalyst.

2. A process according to claim 1, wherein
Az represents a 6-membered heterocyclic group with at least one nitrogen atom, being substituted by one halogen atom and optionally substituted by one C₁-C₆-alkyl or C₁-C₆-haloalkyl group.

3. A process according to claim 1, wherein the acidic catalyt is selected from sulfuric acid, FeCl₃ and ZnCl₂.

4. A process according to claim 1, wherein Az represents an substituted pyridyl group of formula V 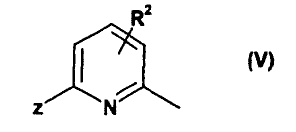 in which
R² represents a hydrogen atom or a C₁-C₆-alkyl or C₁-C₆-haloalkyl group, and
Z represents a halogen atom.

5. A process according to claim 4, wherein the Az is a 6-halopyrid-2-yl group.

6. A process according to claim 4, wherein 1 mole of trichloromethylazine of formula II is treated with 0.4 to 1.2 moles of an acid of formula III.

7. A process according to claim 4, wherein 1 mole of trichloromethylazine of formula II is treated with an acid of formula III in the presence of 0.01 to 0.10 moles of the acidic catalyst.

8. The use of a compound of formula I for the preparation of aryloxyheteroarylcarboxamides of formula VI 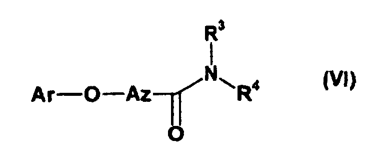 in which
Az has the meaning given in any of the preceding claims, and
Ar represents a phenyl group being substituted by at least one halogen atom or a C₁-C₆-haloalkyl or C₁-C₆-haloalkoxy group,
R³ represents a hydrogen atom or a C₁-C₆-alkyl group, and
R⁴ represents a phenyl group being substituted by at least one halogen atom or a C₁-C₆-haloalkyl or C₁-C₆-haloalkoxy group,
wherein
(a) the mono-halogenated azinyl acid chloride of formula I being prepared from a mono-halogenated azinyltrichloromethane of formula II according to any of the claims 1 to 7,
(b) is reacted with an amine of the formula VII
HNR³R⁴ (VII)
optionally in the presence of an inert solvent and/or a base, and
(c) the resulting mono-halogenated azinylcarboxamide is reacted with an aromatic hydroxyl compound of formula VIII
Ar-OH (VIII)
in which Ar has the meaning given in the precense of a base.

9. The use according to claim 8, in which the halogenated azinyl acid chloride of formula I is reacted with an amie of formula VII without further purification.

## Patentansprüche

1. Verfahren zur Herstellung von Azinylsäurechloridverbindungen der Formel I 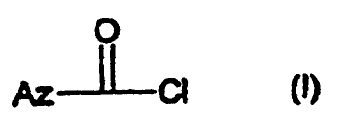 worin
Az für eine 6-gliedrige heterocyclische Gruppe mit mindestens einem Stickstoffatom, die gegebenenfalls durch ein oder mehrere Halogenatome, Nitro, Cyano, C₁-C₆-Alkyl, C₁-C₆-Alkoxy, 4-C₁-C₆-Alkylcyclohexyl oder C₁-C₆-Halogenalkyl substituiert ist, steht,
bei dem man ein Trichlormethylazin der Formel II
Az-CCl₃ (II)
worin Az die angegebene Bedeutung besitzt,
in Gegenwart eines sauren Katalysators mit einer Säure der Formel III
R¹-X-OH (III)
worin
R¹ für C₁-C₆-Alkyl oder C₁-C₆-Halogenalkyl steht und
X für CO oder SO₂ steht,
die das Säurechlorid der Säure der Formel III bildet, welches bei der Umsetzung unter vermindertem Druck abdestilliert werden kann, erhitzt.

2. Verfahren nach Anspruch 1, bei dem
Az für eine 6-gliedrige heterocyclische Gruppe mit mindestens einem Stickstoffatom, die durch ein Halogenatom und gegebenenfalls durch eine C₁-C₆-Alkyl- oder C₁-C₆-Halogenalkylgruppe substituiert ist, steht.

3. Verfahren nach Anspruch 1, bei dem man den sauren Katalysator unter Schwefelsäure, FeCl₃ und ZnCl₂ auswählt.

4. Verfahren nach Anspruch 1, bei dem Az für eine substituierte Pyridylgruppe der Formel V steht 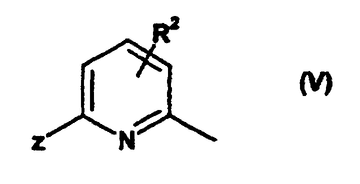 worin
R² für ein Wasserstoffatom oder eine C₁-C₆-Alkyl- oder C₁-C₆-Halogenalkylgruppe steht und
Z für ein Halogenatom steht.

5. Verfahren nach Anspruch 4, bei dem Az für eine 6-Halogenpyrid-2-ylgruppe steht.

6. Verfahren nach Anspruch 4, bei dem man 1 mol Trichlormethylazin der Formel II mit 0,4 bis 1,2 mol einer Säure der Formel III behandelt.

7. Verfahren nach Anspruch 4, bei dem man 1 mol Trichlormethylazin der Formel II in Gegenwart von 0,01 bis 0,10 mol des sauren Katalysators mit einer Säure der Formel III behandelt.

8. Verwendung einer Verbindung der Formel I zur Herstellung von Aryloxyheteroarylcarbonsäureamiden der Formel VI 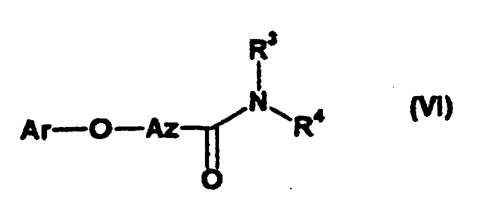 worin
Az die in einem der vorhergehenden Ansprüche angegebene Bedeutung besitzt,
Ar für eine durch mindestens ein Halogenatom oder eine C₁-C₆-Halogenalkyl- oder C₁-C₆-Halogenalkoxygruppe substituierte Phenylgruppe steht,
R³ für ein Wasserstoffatom oder eine C₁-C₆-Alkylgruppe steht und
R⁴ für eine durch mindestens ein Halogenatom oder eine C₁-C₆-Halogenalkyl- oder C₁-C₆-Halogenalkoxygruppe substituierte Phenylgruppe steht,
bei der man
(a) das aus einem einfach halogenierten Azinyltrichlormethan der Formel II nach einem der Ansprüche 1 bis 7 hergestellte einfach halogenierte Azinylsäurechlorid der Formel I
(b) gegebenenfalls in Gegenwart eines inerten Lösungsmittels und/oder einer Base mit einem Amin der Formel VII
HNR³R⁴ (VII)
umsetzt und
(c) das erhaltene einfach halogenierte Azinylcarbonsäureamid in Gegenwart einer Base mit einer aromatischen Hydroxylverbindung der Formel VIII
Ar-OH (VIII)
worin Ar die angegebene Bedeutung besitzt, umsetzt.

9. Verwendung nach Anspruch 8, bei der man das halogenierte Azinylsäurechlorid der Formel I ohne weitere Reinigung mit einem Amin der Formel VII umsetzt.

## Revendications

1. Procédé de préparation de composés de chlorure d'acide azinylique de formule I : 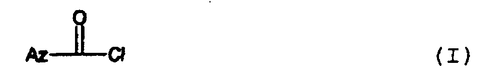 dans laquelle
Az représente un groupe hétérocyclique à 6 chaînons comportant au moins un atome d'azote, qui est éventuellement substitué par un ou plusieurs atomes d'halogène ou groupes nitro, cyano, alkyle en C₁-C₆, alcoxy en C₁-C₆, 4-C₁-C₆-alkyl cyclohexyle ou halogénoalkyle en C₁-C₆,
qui comprend un chauffage d'une trichlorométhylazine de formule II :
Az - CCl₃ (II)
dans laquelle Az a la signification donnée,
avec un acide de formule III :
R¹ - X - OH (III)
dans laquelle
R¹ représente un alkyle en C₁-C₆ ou un halogénoalkyle en C₁-C₆, et
X représente CO ou SO₂,
en présence d'un catalyseur acide,
pour former le chlorure d'acide de l'acide de formule (I) qui peut être distillé pendant la réaction à pression réduite.

2. Procédé suivant la revendication 1, dans lequel
Az représente un groupe hétérocyclique à 6 chaînons comportant au moins un atome d'azote, qui est substitué par un atome d'halogène et est éventuellement substitué par un groupe alkyle en C₁-C₆ ou halogénoalkyle en C₁-C₆.

3. Procédé suivant la revendication 1, dans lequel le catalyseur acide est choisi parmi de l'acide sulfurique, FeCl₃ et ZnCl₂.

4. Procédé suivant la revendication 1, dans lequel Az représente un groupe pyridyle substitué de formule V : 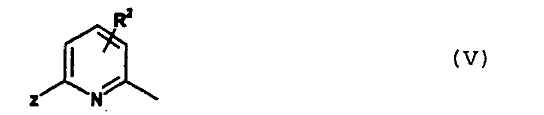 dans laquelle
R² représente un atome d'hydrogène ou un groupe alkyle en C₁-C₆ ou halogénoalkyle en C₁-C₆, et
Z représente un atome d'halogène.

5. Procédé suivant la revendication 4, dans lequel Az est un groupe 6-halogénopyrid-2-yle.

6. Procédé suivant la revendication 4, dans lequel 1 mole de trichlorométhylazine de formule II est traitée par 0,4 à 1,2 mole d'un acide de formule III.

7. Procédé suivant la revendication 4, dans lequel 1 mole de trichlorométhylazine de formule II est traitée par un acide de formule III en présence de 0,01 à 0,10 mole du catalyseur acide.

8. Utilisation d'un composé de formule I pour la préparation d'aryloxyhétéroarylcarboxamides de formule VI : 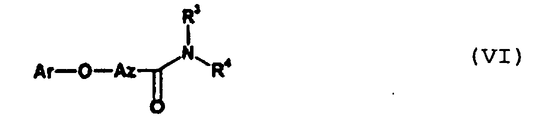 dans laquelle
Az a la signification donnée dans une quelconque des revendications précédentes, et
Ar représente un groupe phényle qui est substitué par au moins un atome d'halogène ou un groupe halogénoalkyle en C₁-C₆ ou halogénoalcoxy en C₁-C₆,
R³ représente un atome d'hydrogène ou un groupe alkyle en C₁-C₆, et
R⁴ représente un groupe phényle qui est substitué par au moins un atome d'halogène ou un groupe halogénoalkyle en C₁-C₆ ou halogénoalcoxy en C₁-C₆,
dans laquelle
(a) le chlorure d'acide azinylique monohalogéné de formule I, qui est préparé à partir d'un azinyltrichlorométhane monohalogéné de formule II suivant l'une quelconque des revendications 1 à 7,
(b) est mis à réagir avec une amine de formule VII :
HNR³R⁴ (VII)
éventuellement en présence d'un solvant inerte et/ou d'une base, et
(c) l'azinylcarboxamide monohalogéné résultant est mis à réagir avec un composé hydroxylé aromatique de formule VIII :
Ar-OH (VIII)
dans laquelle Ar a la signification donnée, en présence d'une base.

9. Utilisation suivant la revendication 8, dans laquelle le chlorure d'acide azinylique halogéné de formule I est mis à réagir avec une amine de formule VII sans autre purification.
